(19) Europäisches Patentamt
European Patent Office
Office européen des brevets

(11) Publication number: **0 171 600 B1**

# (12) EUROPEAN PATENT SPECIFICATION

(45) Date of publication of patent specification: **12.06.91**

(51) Int. Cl.5: **C07D 209/86**, C07D 209/88, C08F 2/50

(21) Application number: **85108524.1**

(22) Date of filing: **09.07.85**

The file contains technical information submitted after the application was filed and not included in this specification

## (54) Carbazole photoinitiators for the photopolymerization of unsaturated compounds.

(30) Priority: **10.07.84 JP 142792/84**

(43) Date of publication of application:
**19.02.86 Bulletin 86/08**

(45) Publication of the grant of the patent:
**12.06.91 Bulletin 91/24**

(84) Designated Contracting States:
**BE CH DE FR GB LI NL**

(56) References cited:
**EP-A- 0 003 002**
**EP-A- 0 116 121**
**EP-A- 0 117 233**
**US-A- 3 946 021**

(73) Proprietor: **ADEKA ARGUS CHEMICAL CO., Ltd.**
**5-2-13 Shirahata, Urawa City**
**Saitama Prefecture(JP)**

(72) Inventor: **Kubota, Naohiro**
**Ageohigashi-danchi 3-105 404-1 Ageomura**
**Ageo City Saitama(JP)**

(74) Representative: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40(DE)**

EP 0 171 600 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

Photochemical polymerization processes have attained substantial importance in the art, especially in those cases where thin layers have to be hardened in a short time, for example, in the hardening of varnish coatings, or in the drying of printing inks.

Many compounds are known as photoinitiators for the photopolymerization of unsaturated compounds. Aromatic ketones such as benzophenone are most commonly used, due to their excellent solubility to unsaturated compounds. Recently, α-substituted aromatic aliphatic ketones were proposed as photoinitiators in U. S. Patent No. 4,308,400, and sulfur-containing-α-amino-aromatic-aliphatic ketones were proposed as photoinitiators in Japan Kokai No. 83-157805.

Many of these known photoinitiators have the shortcoming that when mixed with an unsaturated compound they display an insufficient storage life. Furthermore, these photoinitiators tend to cause an undesirable yellowing of the polymerized composition, and they are insufficiently reactive, as evidenced by a relatively long polymerization time. Some are rapidly inactivated by atmospheric oxygen.

The European Patent Application 0117 233 describes photosetting dyed material containing

a) an olefinically unsaturated photopolymerizable binder,

b) a pigment or a dye and

c) at least one of the compounds I, II or III acting as photoinitiator

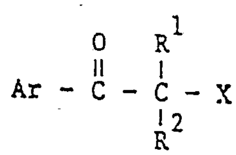

(I)

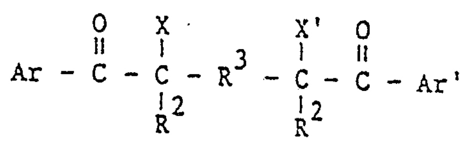

(II)

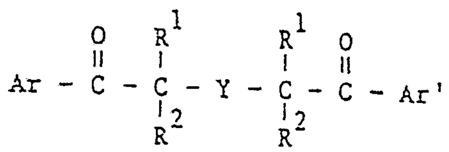

(III),

wherein Ar is an aromatic group containing oxygen, selected from one of the following formulae

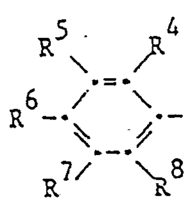

(IV)

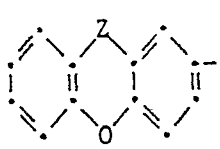

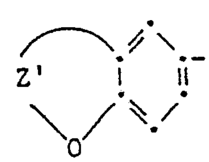

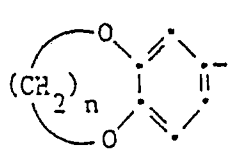

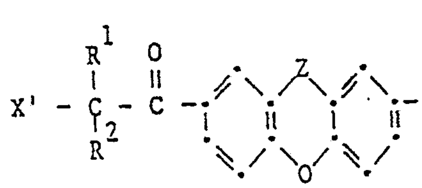

wherein

n

is 1, 2 or 3,

Z

is a direct bond $-CH_2-$, $-CH_2CH_2-$ or -O-,

Z'

is $-CH_2CH_2-$, unsubstituted or substituted by $CH_3$, or $-(CH_2)_3-$,

$R^4$, $R^5$, $R^6$, $R^7$ and $R^8$

independently from each other are hydrogen, halogen, $C_1$-$C_4$-alkyl, $C_3$-$C_{12}$-alkenyl, $C_5$-$C_6$-cycloalkyl, phenyl, -COOH, -COO($C_1$-$C_4$)-alkyl, -OH or $-OR^9$, provided that at least one of the groups $R^4$ to $R^8$ is -OH or $-OR^9$,

$R^9$

means $c_1$-$C_{12}$-alkyl, $C_3$-$C_{12}$-alkenyl, cyclohexyl, hydroxycyclohexyl, $C_1$ -$C_4$-alkyl substituted by one or several of the groups Cl, Br, CN, SH, -N($C_1$-$C_4$-alkyl)$_2$,

piperidino, morpholino, OH, -O($C_1$-$C_4$-alkyl), $-OCH_2$ $CH_2$ CN, $-OCH_2CH_2COO$($C_1$-$C_4$-alkyl), $-OOC-R^{10}$, -COOH, -COO($C_1$-$C_8$-alkyl), -CONH($C_1$-$C_4$-alkyl), -CON($C_1$-$C_4$-alkyl)$_2$,

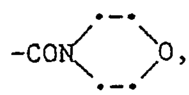

-CO-($C_1$-$C_4$-alkyl) or -CO-phenyl-substituted $C_{1-4}$ alkyl, 2,3-epoxypropyl, $(CH_2CH_2O)_q$-H, phenyl, $C_7$-$C_9$-phenylalkyl, $C_7$-$C_9$-phenylhydroxyalkyl, phenyl substituted by halogen, $C_1$-$C_4$-alkyl, $C_1$-$C_4$-alkoxy or -COO-($C_1$-$C_4$-alkyl); tetrahydropyranyl, tetrahydrofuranyl, a group $-CO-R^{10}$, -COO($C_1$-$C_8$-alkyl), -CONH($C_1$-$C_4$-alkyl) -CON($C_1$-$C_4$-alkyl)$_2$, $-Si(R^{15})(R^{16})_2$, $-SO_2-R^{17}$ or a group with the formula

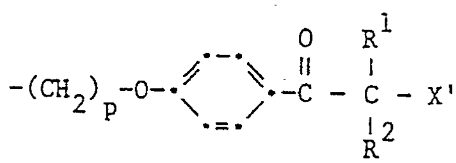 , 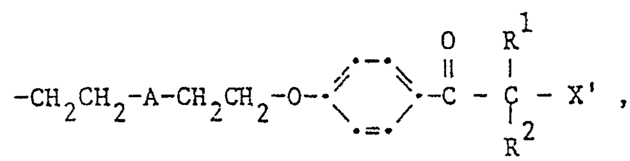

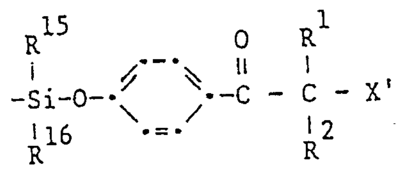 , 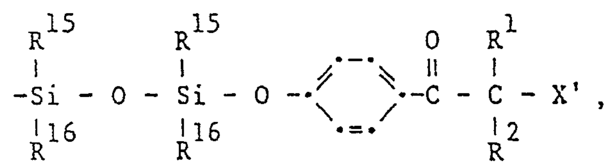

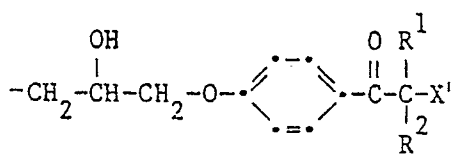

or 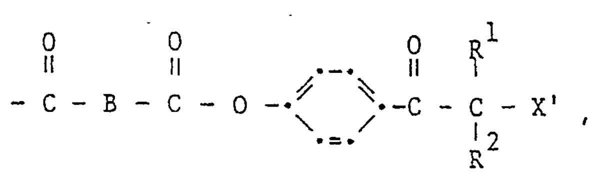

wherein p is 1 to 4, g is 2 to 20, A is oxygen or sulphur and

B

is a direct bond or a $C_1$-$C_{10}$-alkylene group, or

$R^9$

is a group of the formula

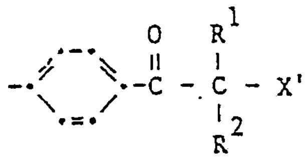 or 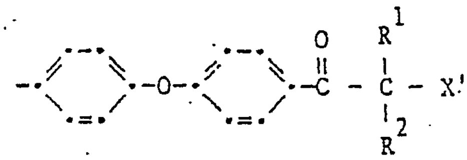 ,

$R^{10}$
is $C_1$-$C_4$-alkyl, $C_2$-$C_4$-alkenyl or phenyl,

X
and X' are amino groups -N($R^{11}$) ($R^{12}$),

Y
means a divalent group of the formula

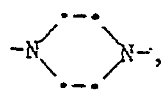

-N($R^{13}$)- or -N($R^{13}$)-$(CH_2)_x$-N($R^{13'}$)-, wherein x is 1 to 8,

$R^{11}$
means hydrogen, $C_1$-$C_{12}$-alkyl, $C_2$-$C_4$-alkyl substituted by one or several of the groups OH, $C_1$-$C_4$-alkoxy, CN or -COO ($C_1$-$C_4$-alkyl); $C_3$-$C_5$-alkenyl, cyclohexyl, $C_7$-$C_9$-phenylalkyl, phenyl or phenyl substituted by Cl, $C_1$-$C_4$-alkyl, OH, $C_1$-$C_4$-alkoxy or -COO($C_1$-$C_4$-alkyl), or $R^{11}$ and $R^1$ together stand for the group $-CH_2OCH_2-$,

$R^{12}$
has one of the meanings given for $R^{11}$ or means together with $R^{11}$ $C_3$-$C_7$-alkylene that can be interrupted by -O-, -S- or -N($R^{14}$ )-, or $R^{12}$ means together with $R^2$ $C_1$-$C_8$-alkylene $C_7$-$C_{10}$-phenylalkylene o-xylylene or $C_2$-$C_4$-oxa- or azaalkylene,

$R^{13}$ and $R^{13'}$
are hydrogen, $C_1$-$C_{12}$-alkyl, $C_1$-$C_4$-hydroxyalkyl, cyclohexyl or benzyl,

$R^{14}$
means hydrogen, $C_1$-$C_4$-alkyl, $C_1$-$C_4$-hydroxyalkyl, $-CH_2CH_2CN$ or $-CH_2CH_2COO$($C_1$-$C_4$-alkyl),

$R^{15}$ and $R^{16}$
mean $C_1$-$C_4$-alkyl or phenyl,

$R^{17}$
means a $C_1$-$R_{18}$-alkyl, phenyl or $C_7$-$C_{20}$-alkylphenyl,

Ar'
has one of the meanings given for Ar,

$R^1$ and $R^2$
independently from each other are $C_1$-$C_8$-alkyl, $C_1$-$C_4$-alkyl substituted by OH, $C_1$-$C_4$ -alkoxy, CN, -COO-($C_1$-$C_8$-alkyl) or N($R^{11}$) ($R^{12}$); allyl, phenyl, chlorophenyl,$R^9$-O-phenyl or $C_7$-$C_9$-phenylalkyl or $R^1$ and $R^2$ together mean $C_2$-$C_8$-alkylene $C_3$-$C_9$-oxa- or azaalkylene.

$R^3$
is a direct bond, $C_1$-$C_6$-alkylene, $C_2$-$C_6$-oxaalkylene or cyclohexylene or forms together with both substituents $R^2$ and both C-atoms which those substituents are bond to, a cyclopentane-, cyclohexane-, cyclohexene-, endomethylencyclohexane-or endometylencyclohexene ring, or a salt of such compound formed by acid addition.

Furthermore these compounds used as photoinitiators are claimed.

The US-A-3 946 021 discloses bis-basic ketones of carbazole having the following formula:

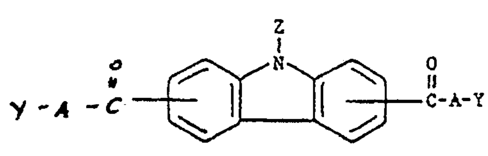

wherein Z is hydrogen or straight or branched lower alkyl having from 1 to 4 carbon atoms; A is a straight or branched alkylene chain having from 1 to about 6 carbon atoms; and each Y is

A. the group

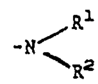

wherein $R^1$ and $R^2$ are individually hydrogen or lower alkyl having from 1 to about 4 carbon atoms; or
B. the group

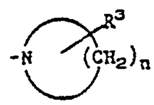

wherein n is a whole integer of 4 or 5, and $R^3$ is hydrogen or lower alkyl having from 1 to about 4 carbon atoms and can be linked to any one of the carbon atoms of the heterocyclic group; or
C. the group

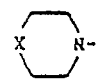

wherein X is oxygen or $NR^4$, and $R^4$ is hydrogen or lower alkyl of from 1 to about 4 carbon atoms; or a pharmaceutically acceptable acid addition salt of said base.

It is stated that this compounds have antiviral activity.

There is therefore a need in the art for photoinitiators which are readily soluble in the substrate, and which, having a good storage life in the dark, initiate the photopolymerization more rapidly and give a higher polymer yield per unit of time, than the known photoinitiators.

In accordance with this invention, carbazole photoinitiators are provided having the following formulae (I), (II) and (III) that possess the required properties as photoinitiators. In particular, they effect a rapid photopolymerization, and either do not display the shortcomings referred to, or display them to a much lesser degree, than the known photoinitiators. Furthermore, they are suitable for the photochemical cross linking of polyolefins.

Subject matter of this invention is a photopolymerizable prepolymer composition comprising carbazole photoinitiator which has one of the formulae:

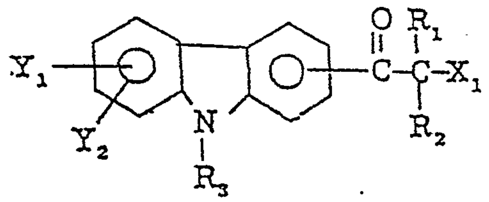

(I)

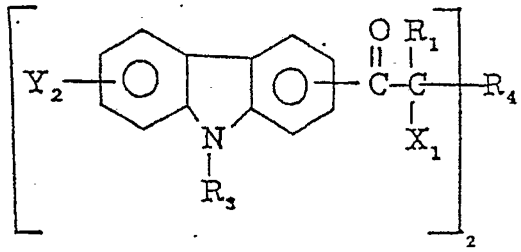

(II)

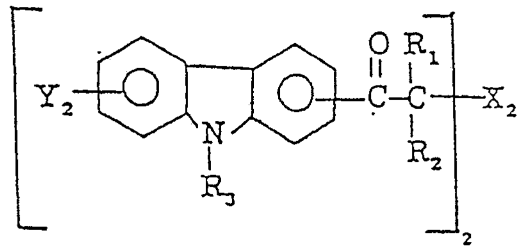

(III)

wherein:

$R_1$ and $R_2$ are each alkyl having from 1 to 18 carbon atoms; or are taken together to form alkylene having from 2 to 12 carbon atoms;

$R_3$ is selected from the group consisting of hydrogen; alkyl having from 1 to 18 carbon atoms; and acyl having from 2 to 12 carbon atoms;

$X_1$ is $-OR_5$ or 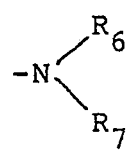

in which $R_5$ is selected from the group consisting of hydrogen; alkyl and alkenyl having from 1 to 18 carbon atoms; and $R_6$ and $R_7$ are selected from the group consisting of alkyl; hydroxyalkyl having from 1 to 18 carbon atoms; and $R_6$ and $R_7$ taken together to form alkylene having from 2 to 12 carbon atoms; and oxadialkylene and iminodialkylene having from 4 to 24 carbon atoms;

$Y_1$ is hydrogen or 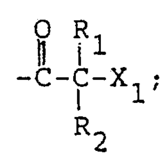 ;

$Y_2$ is selected from the group consisting of hydrogen, halogen and nitro;

$R_4$ is a direct linkage (-) or alkylene having from 1 to 12 carbon atoms;

$X_2$ is $-O-R_8-O-$ or 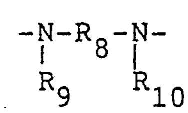

in which:

$R_8$ is alkylene having from 2 to 12 carbon atoms;
$R_9$ and $R_{10}$ are alkyl having from 1 to 18 carbon atoms; or are taken together to form alkylene having from 1 to 12 carbon atoms;
and an unsaturated compound having at least one ethylenic double bond that is photopolymerizable to form a polymer.

Exemplary $R_1$, $R_2$, $R_3$, $R_5$, $R_6$, $R_7$, $R_9$ and $R_{10}$ alkyl are methyl, ethyl, propyl, isopropyl, butyl, sec-butyl, t-butyl, isobutyl, amyl, sec-amyl, isoamyl, tert-amyl, hexyl, isohexyl, sec-hexyl, tert.-hexyl, heptyl, isoheptyl, tert.-heptyl, sec.-heptyl, octyl, isooctyl, 2-ethylhexyl, nonyl, isononyl, tert.-nonyl, decyl, undecyl, dodecyl, tridecyl, tetradecyl, heptadecyl and octadecyl.

Exemplary $R_1$ and R2 taken together as alkylene include ethylene, propylene, butylene, amylene, hexylene,heptylene, octylene, nonylene, decylene and dodecylene.

Exemplary $R_3$ acyl include acetyl, propionyl, butyroyl, valeroyl, caproyl, 2-ethylhexanoyl, decanoyl and lauroyl; acryloyl, methacryloyl, vinylacetyl, benzoyl, toluoyl, t-butylbenzoyl and chlorobenzoyl.

Exemplary $R_5$ alkenyl include allyl and octadecenyl.

Exemplary $R_6$ and $R_7$ hydroxyalkyl include hydroxyethyl, 2-hydroxypropyl and 2-hydroxybutyl, 2-hydroxyhexyl, 2-hydroxyoctyl, 2-hydroxynonyl, and 2-hydroxydodecyl.

Exemplary $R_6$ and $R_7$ alkylene, oxadialkylene and iminodialkylene include ethylene, propylene, butylene, amylene, hexylene, heptylene, octylene, decylene and dodecylene, oxadiethylene and iminodiethylene.

Exemplary $Y_2$ halogen include chlorine, bromine, fluorine and iodine.

Exemplary $R_4$ alkylene include methylene, ethylene, trimethylene, tetramethylene, hexamethylene, octamethylene, decamethylene and dodecamethylene.

Exemplary $R_8$ alkylene include ethylene, propylene, 2, 2-dimethylpropylene, 1,4-butylene, hexamethylene and decamethylene.

Exemplary $R_9$ and $R_{10}$ alkylene include methylene, ethylene, propylene, butylene, amylene, hexylene, octylene, decylene and dodecylene.

A further subject matter of this invention are carbazole photoinitiator for the photopolymerization of unsaturated compounds of the formulae (I), (II) and (III) shown above.

Special embodiments of the carbazole photoinitiators are those in which:
$R_1$ and $R_2$ are each alkyl having from 1 to 18 carbon atoms,
$R_1$ and $R_2$ are taken together to form alkylene having from 2 to 12 carbon atoms,
$R_3$ is hydrogen,
$R_3$ is alkyl having from 1 to 18 carbon atoms or
$R_3$ is acyl having from 2 to 12 carbon atoms.

The following are carbazole compounds of the formulae (I), (II) and (III) falling within the invention.

1. 3-(2-methyl-2-dimethylaminopropionyl) carbazole
2. 3-(2-methyl-2-morpholinopropionyl)-9-methylcarbazole
3. 3,6-bis(2-methyl-2-morpholinopropionyl)-9-methylcarbazole
4. 3-(2-methyl-2-morpholinopropionyl)-9-butylcarbazole
5. 3-(2-ethyl-2-piperidinopropionyl)-9-butylcarbazole
6. 3-(2-methyl-2-diethanolaminopropionyl)-9-methylcarbazole
7. 3-(2-methyl-2-dibutylaminopropionyl)-6-chlorocarbazole
8. 3-(2-methyl-2-piperazinopropionyl-6-nitro-9-methylcarbazole
9. 3-(2-methyl-2-morpholinopropionyl)-9-acetylcarbazole
10. 3,6-bis(2-methyl-2-morpholinopropionyl)-9-benzoylcarbazole
11. 3-(2-methyl-2-hydroxypropionyl)-9-methylcarbazole
12. 3-(2-methyl-2-methoxypropionyl)-9-methylcarbazole
13. 3-(2-methyl-2-allyloxypropionyl)-9-methylcarbazole
14. 3-(1-hydroxycyclohexanoyl)-9-butylcarbazole
15. 1,4-bis(1-(9-methyl-3-carbazolyloyl)isopropyl)piperazine
16. N,N'-dimethyl-N,N'-bis(1-(9-methyl-3-carbazolyloyl) isopropyl)ethylenediamine.

The carbazole compounds of formulae (I), (II) and (III) can be readily prepared by known procedures, for example, by the method described in US-patent No. 4,308,400.

The compounds of formulae (I) and (III) can be prepared by brominating the compound shown below, reacting the resulting compound with $H-X_1$ or $H-X_2-H$:

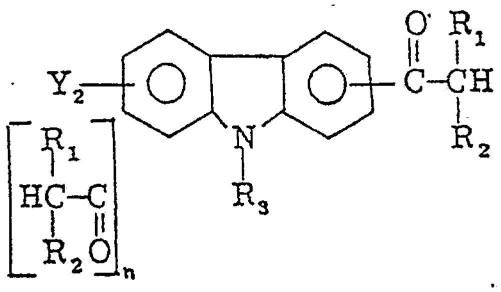

(n= 0 or 1)

The compounds of the formula (II) can be prepared by brominating the compound shown below, reacting the resulting compound with H-$X_1$.

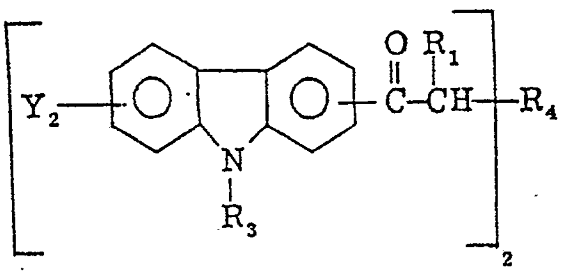

The following example is illustrative:

EXAMPLE I

Synthesis of 3-(2-methyl-2-morpholinopropionyl)-9-methylcarbazole

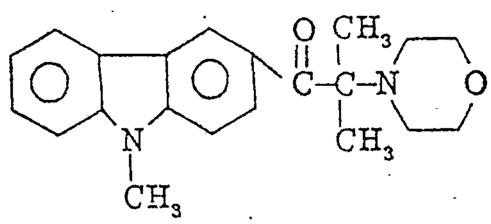

3-(2-Methylpropionyl)-9-methylcarbazole 15 g was dissolved in 50 ml of carbon tetrachloride. A solution of 12 g of bromine in 30 ml of carbon tetrachloride was added dropwise over one hour at room temperature, and the reaction mixture then stirred for one additional hour at room temperature. Then nitrogen gas was introduced to remove the hydrogen bromide produced and excess bromine

Morpholine 30 g was added to the reaction mixture, which was then heated up to 120 °C, while carbon tetrachloride was distilled off. The solution was stirred for 3 hours at 120 °C, and the morpholine hydrobromide filtered out, and excess morpholine distilled off.

Toluene 100 ml was added, and the solution was washed with water, and then extracted with aqueous 3 N HCl solution. The HCl solution was neutralized by adding 3 N NaOH solution, and then extracted with toluene. The toluene solution was washed with water, and then toluene was distilled off. The residue was recrystallized from ethanol. The desired product, melting at 164-166 °C, was obtained.

If desired, the photoinitiators of this invention can be combined with secondary and tertiary alkyl and hydroxyalkyl amines, which act as accelerators.

Such amines include, for example, triethanolamine, triisopropanolamine, methyl-diethanolamine, octyl-diethanolamine, dodecyl-diethanolamine, octadecyl-diethanolamine, dibutylethanolamine, dioctyl-ethanolamine, methyl-hydroxyhexyloctanolamine, diethanolaniline, diethanolamine, methyl-ethanolamine, butyl-ethanolamine, dodecyl-ethanolamine, tetrahydroxyethyl-hexamethylenediamine, triethylamine, tributylamine, dimethylaminopropylamine, dimethylaniline, diethylamine, dibutylamine, dioctylamine, tetramethyl-ethylenediamine and Michler's ketone. Among these amines, the alkanolamines are preferable,

due to their excellent effect.

The photoinitiators of this invention can polymerize or harden any unsaturated compounds containing at least one ethylenic double bond in the molecule, as monomers or prepolymers, by actinic light.

Such unsaturated compounds include, for example, unsaturated carboxylic acids, such as acrylic acid, methacrylic acid, itaconic acid, crotonic acid and maleic acid; esters or amides of such unsaturated carboxylic acids with mono- or polyhydric alcohols or amines; methacrylates of polyhydroxyesters obtained from polyol and polycarboxylic acid; unsaturated polyesters or polyamides obtained from unsaturated dicarboxylic acids and polyols or polyamines; urethane acrylates obtained from urethane compounds containing isocyanate groups prepared from diisocyanates with polyols and hydroxyalkyl methacrylates; epoxyacrylates obtained from polyglycidyl ethers of bisphenols or polyols and methacrylic acid; polyesters obtained from glycidyl methacrylate and dicarboxylic acid; dimethacryl modified polyesters or polyamides obtained from polyesters or polyamides containing carboxyl groups and glycidyl methacrylate.

The unsaturated esters can be derived from mono- or polyhydric alcohols having from one to six hydroxyl groups and from one to eighteen carbon atoms, in an open aliphatic chain or carboxylic or heterocyclic ring, including methanol, ethanol, butanol, allyl alcohol, octanol, ethylene glycol, diethylene glycol, triethylene glycol, propylene glycol, neopentyl glycol, 1,4-butanediol, 1,3-butanediol, 1,6-hexanediol, glycerine, trimethylolpropane, trimethylolethane, tris(2-hydroxyethyl) isocyanurate, pentaerythritol, diglycerine, ditrimethylolpropane and dipentaerythritol; mono- or poly-amines include ammonia, methyl amine, butylamine.

The unsaturated amides can be derived from mono or polyamino amines having from one to six amino groups and from one to eighteen carbon atoms in an open aliphatic chain or carboxylic or heterocyclic ring, including octylamine, diethylamine, dibutylamine, ethylenediamine, diethylenetriamine, piperazine, 1,6-hexamethylenediamine and melamine.

Preferred examples of esters and amides of unsaturated carboxylic acids include methyl-, ethyl-, butyl-, isooctyl- and 2-hydroxyethyl-acrylate, methyl- and ethyl-methacrylate, ethylene glycol diacrylate, triethylene glycol diacrylate, 1,4-butanediol diacrylate, trimethylolpropane triacrylate, pentaerythritol triacrylate, pentaerythritol tetraacrylate, tris-(2-hydroxyethyl) isocyanurate triacrylate, dipentaerythritol tetraacrylate; dipentaerythritol, hexaacrylate, glycerine diacrylate, triethylene glycol dimethacrylate, pentaerythritol trimethacrylate, ethylene glycol dicrotonate, diallyl fumarate bis(acryloyloxyethylphenyl)-propane, acrylamide, methacrylamide, ethylene-bis-(acrylamide), hexamethylene-bis(acrylamide) and methylene-bis-(acrylamide).

Examples of other photopolymerizable compounds are diallyl phthalate, diallyl malonate, divinyl phthalate, vinyl acetate, isobutyl vinyl ether, ethylene glycol divinyl ether, styrene, acrylonitrile, triallyl isocyanurate and triallyl phosphate.

The photoinitiators of this invention are effective in small amounts, and the amount is not critical, but is selected to give the desired rate of photopolymerization according to the unsaturated compound used. Good results are obtained in amounts within the range from about 0.1 to about 20 parts by weight, based on 100 parts of above unsaturated compound. More than this can be used.

The photopolymerizable systems comprising the photoinitiator and unsaturated compound can contain other conventional additives, such as heat polymerization inhibitors, colorants or pigments, plasticizers, surface-protecting agents, and lubricants.

Heat-polymerization inhibitors include hydroquinone, p-methoxyphenol, pyrogallol, catechol, 2, 6-di-t-butyl-p-cresol, $\beta$-naphthol, and t-butylhydroquinone.

Colorants or pigments include carbon black, metal (silver, aluminum) powder, chrome yellow, titanium white, talc, alumina, Milori Blue, Chrome Vermillion, Hansa yellow, Benzidine Yellow, Vulcan Orange, Permanent Orange, Lake Red C, Brilliant Carmine B, Rhodamin Lake, Eosine, Victoria Blue Lake, Phthalocyanine Blue, Phthalocyanine Green, Quinacridone Red, Dioxazine Violet and Methyl Violet.

The polymerization is carried out in the usual way, by irradiation with light. Suitable light sources are, for example, carbon arc, mercury high pressure lamp, xenon lamp, metal halide lamp, tungsten lamp, argon laser and helium-cadmium laser.

The following Examples represent preferred embodiments of photopolymerization systems utilizing photoinitiators of formulae (I), (II) and (III). All parts indicated are by weight. The control compound 2 is known from EP-A-0 003 002.

## Examples 1 to 8

A resin mixture composed of 75 parts of NK ester U-108A (urethane acrylate (m.w. = 1500) available from Shinnakamura Kagaku), 25 parts of 1,6-hexanediol diacrylate and 0.5 part of photoinitiator as indicated

in Table I was applied to paper using a film-drawing device to a thickness of 50 mμ. The films were irradiated with a mercury high pressure lamp (Toshiba; Model H-400P) for 15 seconds (distance = 12 cm). The pencil hardness of the hardened films was measured, and the results are shown in Table I.

Table I

| Example No. | Photoinitiator | Pencil hardness |
|---|---|---|
| Control 1 | Benzophenone | 6B |
| Control 2 | 1-(2-methyl-2-morpholino-propionyl)-4-methylthiobenzene | 3H |
| Example 1 | 3-(2-methyl-2-dimethylamino-propionyl) carbazole | 8H |
| Example 2 | 3-(2-methyl-2-morpholino-propionyl)-9-methylcarbazole | 9H |
| Example 3 | 3-(2-methyl-2-morpholino-propionyl)-9-butylcarbazole | 9H |
| Example 4 | 3-(2-methyl-2-dibutylamino-propionyl)-6-chlorocarbazole | 7H |
| Example 5 | 3-(2-methyl-2-morpholino-propionyl)-9-acetylcarbazole | 6H |
| Example 6 | 3-(2-methyl-2-methoxypropionyl)-9-methylcarbazole | 7H |
| Example 7 | 3-(1-hydroxycyclohexanoyl)-9-butylcarbazole | 8H |

Table I (continued)

| Example No. | Photoinitiator | Pencil hardness |
|---|---|---|
| Example 8 | 2,7-bis(1-(9-butyl-3-carbazolyloyl) isopropyl)-2,7-dimethyl-3,6-dioxaoctanol | 7H |

The superior hardness when the photoinitiator of the invention was used is apparent from the results.

Examples 9 to 16

A resin mixture composed of 80 parts of NK-ester UVX-2(epoxy acrylate (m.w. = 1500) available from Shinnakamura Kagaku), 20 parts of trimethylolpropane triacrylate, 70 parts of $TiO_2$, 6 parts of N-methyldiethanolamine and 6 parts of photoinitiator as indicated in Table II was applied to paper using a film-drawing device to a thickness of 10 mμ. These films were irradiated with a mercury high pressure lamp (Toshiba; Model H-400P) for 30 seconds (distance = 15 cm). The pencil hardness of the hardened films was then measured, and the results are shown in Table II.

Table II

| Example No. | Photoinitiator | Pencil hardness |
|---|---|---|
| Control 1 | Benzophenone | 5B |
| Control 2 | 1-(2-methyl-2-morpholino propionyl)-4-methylthiobenzene | HB |
| Example 9 | 3-(2-methyl-2-morpholino-propionyl)-9-methylcarbazole | 9H |
| Example 10 | 3,6-bis(2-methyl-2-morpholino-propionyl)-9-methylcarbazole | 8H |
| Example 11 | 3-(2-methyl-2-morpholino-propionyl)-9-butylcarbazole | 9H |
| Example 12 | 3-(2-methyl-2-diethanolamino-propionyl)-9-methylcarbazole | 8H |
| Example 13 | 3,6-bis(2-methyl-2-morpholino-propionyl)-9-benzoylcarbazole | 7H |
| Example 14 | 3-(2-methyl-2-allyloxypropionyl)-9-methylcarbazole | 6H |
| Example 15 | 1,4-bis(1-(9-methyl-3-carbazolyloyl) isopropyl) piperidine | 8H |
| Example 16 | N,N'-dimethyl-N,N'-bis(1-(9-methyl-3-carbazolyloyl) isopropyl) ethylenediamine | 7H |

The superior hardness when the photoinitiator of the invention was used is apparent from the results.

Examples 17 to 23

A resin mixture composed of 80 parts of NK-ester U-108A (urethane acrylate (m.w. = 1500) available

from Shinnakamura Kagaku), 20 parts of trimethylolpropane triacrylate, 22 parts of phthalocyanine blue (Dainichiseika; SR-5020), 7 parts of ethyl-4-dimethylaminobenzoate and 7 parts of photoinitiator as indicated in Table III was applied to paper using a film-drawing device to a thickness of 10 mμ. These films were irradiated with a mercury high pressure lamp (Toshiba; Model H-400P) for 30 seconds (distance = 15 cm). The pencil hardness of the hardened films was measured, and the results are shown in Table III.

Table III

| Example No. | Photoinitiator | Pencil hardness |
|---|---|---|
| Control 1 | Benzophenone | 2B |
| Control 2 | 1-(2-methyl-2-morpholino-propionyl)-4-methylthiobenzene | 2H |
| Example 17 | 3-(2-methyl-2-dimethylamino-propionyl) carbazole | 6H |
| Example 18 | 3-(2-methyl-2-morpholino-propionyl)-9-methylcarbazole | 7H |
| Example 19 | 3-(2-methyl-2-morpholino-propionyl)-9-butylcarbazole | 7H |
| Example 20 | 3-(2-ethyl-2-piperidino-propionyl)-9-butylcarbazole | 6H |
| Example 21 | 3-(2-methyl-2-piperazinopropionyl-6-nitro-9-methylcarbazole | 5H |

Table III (continued)

| Example No. | Photoinitiator | Pencil hardness |
|---|---|---|
| Example 22 | 3-(2-methyl-2-hydroxy-propionyl)-9-methylcarbazole | 6H |
| Example 23 | 1,4-bis(1-(9-methyl-3-carbazolyloyl) isopropyl) piperidine | 5H |

The superior hardness when the photoinitiator of the invention was used is apparent from the results.

**Claims**

**1.** A photopolymerizable prepolymer composition comprising a carbazole photoinitiator which has one of the formulae:

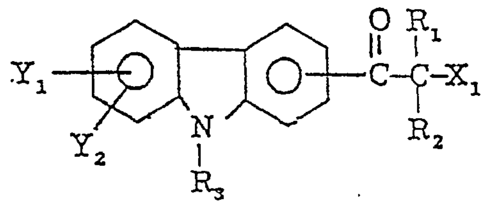

(I)

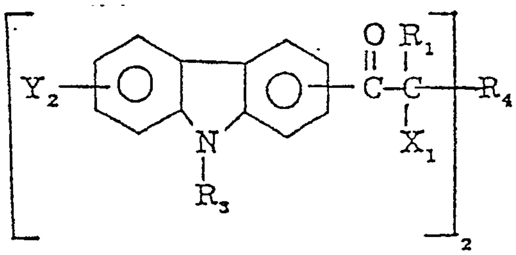

(II)

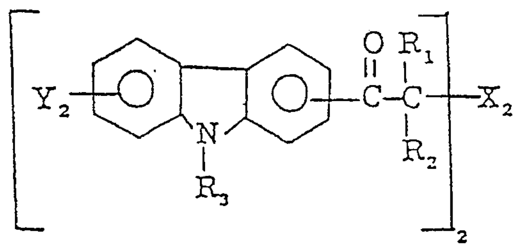

(III)

wherein:

$R_1$ and $R_2$ are each alkyl having from 1 to 18 carbon atoms; or are taken together to form alkylene having from 2 to 12 carbon atoms;

$R_3$ is selected from the group consisting of hydrogen; alkyl having from 1 to 18 carbon atoms; and acyl having from 2 to 12 carbon atoms;

$X_1$ is $-OR_5$ or 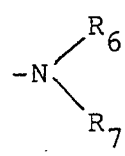

in which $R_5$ is selected from the group consisting of hydrogen; alkyl and alkenyl having from 1 to 18 carbon atoms; and $R_6$ and $R_7$ are selected from the group consisting of alkyl; hydroxyalkyl having from 1 to 18 carbon atoms; and $R_6$ and $R_7$ taken together to form alkylene having from 2 to 12 carbon atoms; and oxadialkylene and iminodialkylene having from 4 to 24 carbon atoms; $Y_1$ is hydrogen or

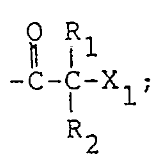;

$Y_2$ is selected from the group consisting of hydrogen, halogen and nitro;
$R_4$ is a direct linkage (-) or alkylene having from 1 to 12 carbon atoms;
$X_2$ is $-O-R_8-O-$ or

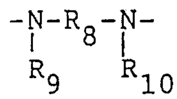

in which:
$R_8$ is alkylene having from 2 to 12 carbon atoms; $R_9$ and $R_{10}$ are alkyl having from 1 to 18 carbon atoms; or are taken together to form alkylene having from 1 to 12 carbon atoms;
and an unsaturated compound having at least one ethylenic double bond that is photopolymerizable to form a polymer.

2. A photopolymerizable prepolymer composition comprising a photoinitiator according to claim 1 also containing a secondary or tertiary alkyl or hydroxyalkyl amine.

3. A process for photopolymerizing an unsaturated compound having at least one photopolymerizable ethylenic double bond, which comprises initiating the unsaturated compound with actinic light in the presence of a photoinitiator as defined in claim 1.

4. Carbazole photoinitiators for the photopolymerization of unsaturated compounds, having one of the formulae:

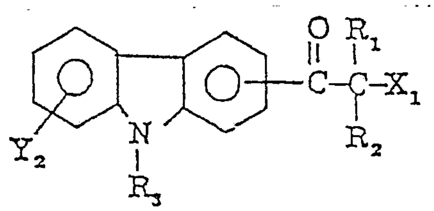
(I)

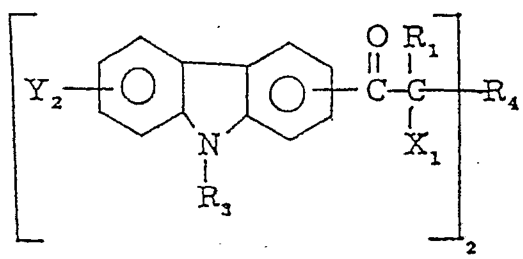
(II)

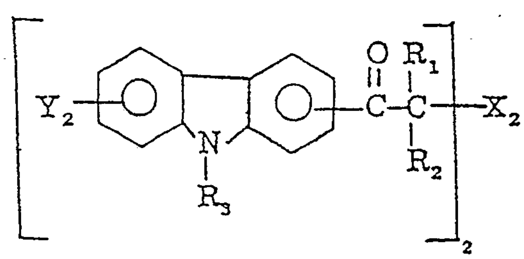
(III)

wherein:
$R_1$ and $R_2$ are each alkyl having from 1 to 18 carbon atoms; or are taken together to form alkylene having from 2 to 12 carbon atoms;

$R_3$ is selected from the group consisting of hydrogen; alkyl having from 1 to 18 carbon atoms; and acyl having from 2 to 12 carbon atoms;
$X_1$ is $-OR_5$ or

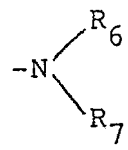

in which $R_5$ is selected from the group consisting of hydrogen; alkyl and alkenyl having from 1 to 18 carbon atoms; and $R_6$ and $R_7$ are selected from the group consisting of alkyl; hydroxyalkyl having from 1 to 18 carbon atoms; and $R_6$ and $R_7$ taken together to form alkylene having from 2 to 12 carbon atoms; and oxadialkylene and iminodialkylene having from 4 to 24 carbon atoms; $Y_2$ is selected from the group consisting of hydrogen, halogen and nitro;
$R_4$ is a direct linkage (-) or alkylene having from 1 to 12 carbon atoms;
$X_2$ is -O-$R_8$-O- or

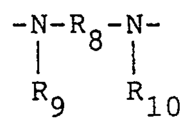

in which:
$R_8$ is alkylene having from 2 to 12 carbon atoms; $R_9$ and $R_{10}$ are alkyl having from 1 to 18 carbon atoms; or are taken together to form alkylene having from 1 to 12 carbon atoms.

5. Carbazole photoinitiators according to claim 4 in which $R_1$ and $R_2$ are each alkyl having from 1 to 18 carbon atoms.

6. Carbazole photoinitiators according to claim 4 in which $R_1$ and $R_2$ are taken together to form alkylene having from 2 to 12 carbon atoms.

7. Carbazole photoinitiators according to claim 4 in which $R_3$ is hydrogen.

8. Carbazole photoinitiators according to claim 4 in which $R_3$ is alkyl having from 1 to 18 carbon atoms.

9. Carbazole photoinitiators according to claim 4 in which $R_3$ is acyl having from 2 to 12 carbon atoms.

**Revendications**

1. Une composition de prépolymère photopolymérisable comprenant un photo-inducteur du type carbazole qui répond a l'une des formules

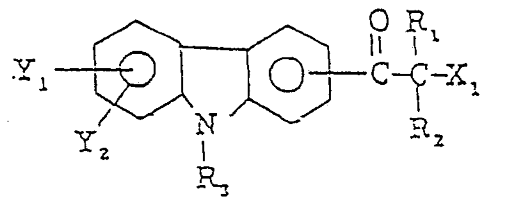

(I)

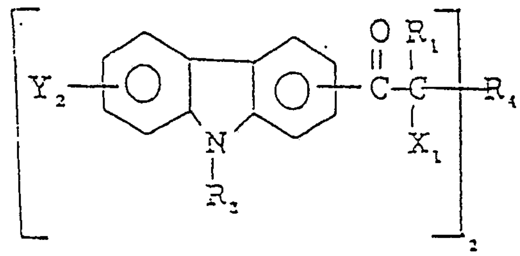

(II)

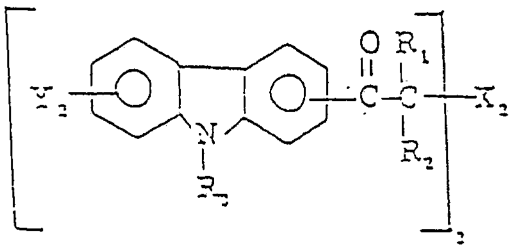

(III)

dans lesquelles
$R_1$ et $R_2$ sont chacun un groupe alkyle en $C_1$-$C_{18}$ ou bien, pris ensemble, ils forment un groupe alkylène en $C_2$-$C_{12}$;
$R_3$ est choisi parmi l'hydrogène, un groupe alkyle en $C_1$-$C_{18}$ et un groupe acyle en $C_2$-$C_{12}$;
$X_1$ est un groupe de formule $-OR_5$ ou

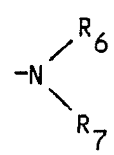

dans lesquelles $R_5$ est choisi parmi l'hydrogène et les groupes alkyles et alcényles en $C_1$-$C_{18}$ ; et $R_6$ et $R_7$ sont choisis parmi les groupes alkyles et hydroxyalkyles en $C_1$-$C_{18}$ ; ou bien $R_6$ et $R_7$, pris ensemble, forment un groupe alkylène en $C_2$-$C_{12}$ ou oxadialkylène ou iminodialkylène en $C_4$-$C_{24}$ ;
$Y_1$ est un atome d'hydrogène ou un groupe

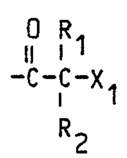 ;

$Y_2$ est choisi parmi l'hydrogène, un halogène et un groupe nitro ;
$R_4$ est une liaison directe (-) ou un groupe alkylène en $C_1$-$C_{12}$ ;
$X_2$ est $-O-R_8-O-$ ou

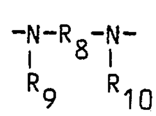

où
$R_8$ est un groupe alkylène en $C_2$-$C_{12}$ ;
$R_9$ et $R_{10}$ sont chacun un groupe alkyle en $C_1$-$C_{18}$ ou bien, pris ensemble, ils forment un groupe alkylène en $C_1$ -$C_{12}$ ;
et un composé insaturé ayant au moins une double liaison éthylénique qui est photopolymérisable pour former un polymère.

2. Une composition de prépolymère photopolymérisable comprenant un photo-inducteur selon la revendication 1, contenant également une alkyl- ou hydroxyalkylamine seondaire ou tertiaire.

3. Un procédé pour la photopolymérisation d'un composé insaturé ayant au moins une double liaison éthylénique photopolymérisable, qui consiste à activer le composé insaturé par la lumière actinique en présence d'un photo-inducteur tel que défini à la revendication 1.

4. Photo-inducteurs du type carbazole pour la photopolymérisation de composés insaturés, répondant à l'une des formules :

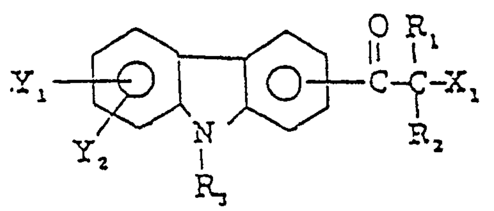
(I)

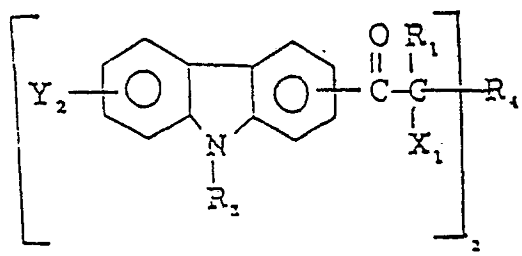
(II)

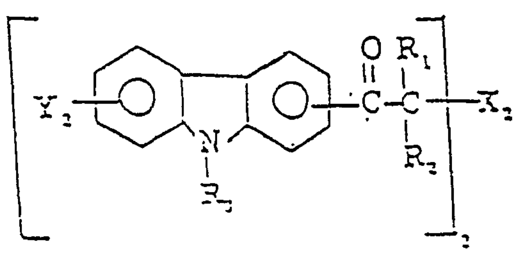
(III)

dans lesquelles
$R_1$ et $R_2$
sont chacun un groupe alkyle en $C_1$-$C_{18}$ ; ou bien, pris ensemble, ils forment un groupe alkylène en $C_2$-$C_{12}$ ;
$R_3$
est choisi parmi l'hydrogène, un groupe alkyle en $C_1$-$C_{18}$ et un groupe acyle en $C_2$-$C_{12}$ ;
$X_1$
est un groupe de formule -$OR_5$ ou

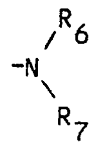

dans lesquelles $R_5$ est choisi parmi l'hydrogène et les groupes alkyles et alcényles en $C_1$-$C_{18}$ ; et $R_6$ et $R_7$ sont choisis parmi les groupes alkyles et hydroxyalkyles en $C_1$-$C_{18}$ ; ou bien $R_6$ et $R_7$ pris ensemble forment un groupe alkylène en $C_2$-$C_{12}$ ou oxadialkylène ou iminodialkylène en $C_4$-$C_{24}$ ;

$Y_2$

est choisi parmi l'hydrogène, un halogène et un groupe nitro ;

$R_4$

est une liaison directe (-) ou un groupe alkylène en $C_1$-$C_{12}$ ;

$X_2$

est -O-$R_8$-O- ou

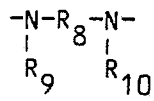

où

$R_8$ est un groupe alkylène en $C_2$-$C_{12}$ ;

$R_9$ et $R_{10}$ sont chacun un groupe alkyle en $C_1$ -$C_{18}$ ou bien, pris ensemble, ils forment un groupe alkylène en $C_1$-$C_{12}$.

**5.** Photo-inducteurs du type carbazole selon la revendication 4, dans lesquels $R_1$ et $R_2$ sont chacun un groupe alkyle en $C_1$-$C_{18}$.

**6.** Photo-inducteurs du type carbazole selon la revendi-cation 4, dans lesquels $R_1$ et $R_2$ sont pris ensemble pour former un groupe alkylène en $C_2$-$C_{12}$.

**7.** Photo-inducteurs du type carbazole selon la revendication 4, dans lesquels $R_3$ est l'hydrogène.

**8.** Photo-inducteurs du type carbazole selon la revendication 4, dans lesquels $R_3$ est un groupe alkyle en $C_1$-$C_{18}$.

**9.** Photo-inducteurs du type carbazole selon la revendication 4, dans lesquels $R_3$ est un groupe acyle en $C_2$-$C_{12}$.

## Ansprüche

**1.** Photopolymerisierbare Prepolymer-Zusammensetzung, die umfaßt: einen Carbazolphotoinitiator mit einer der Formeln:

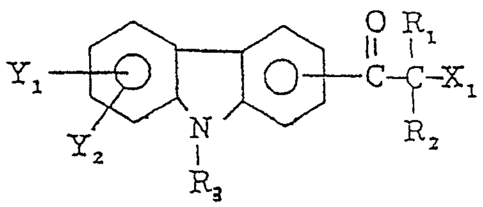

(I)

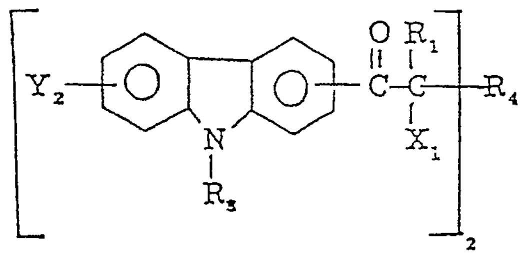

(II)

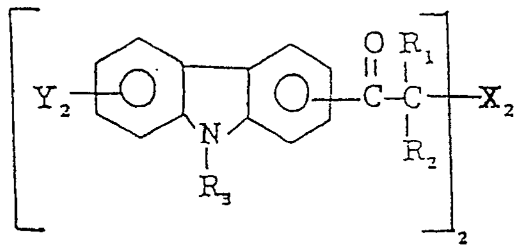

(III)

worin

$R_1$ und $R_2$ jeweils Alkyl mit 1 bis 18 Kohlenstoffatomen bedeuten oder, zusammen genommen, Alkylen mit 2 bis 12 Kohlenstoffatomen bilden,

$R_3$ aus der aus Wasserstoff, Alkyl mit 1 bis 18 Kohlenstoffatomen und Acyl mit 2 bis 12 Kohlenstoffatomen bestehenden Gruppe ausgewählt ist,

$X_1$ $-OR_5$ oder

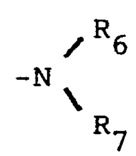

-N ist, worin $R_5$ aus der aus Wasserstoff, Alkyl und Alkenyl mit 1 bis 18 Kohlenstoffatomen bestehenden Gruppe ausgewählt ist und $R_6$ und $R_7$ aus der aus Alkyl, Hydroxyalkyl mit 1 bis 18 Kohlenstoffatomen bestehenden Gruppe ausgewählt sind, und $R_6$ und $R_7$, zusammen genommen, Alkylen mit 2 bis 12 Kohlenstoffatomen und Oxadialkylen und Iminodialkylen mit 4 bis 24 Kohlenstoffatomen bilden,

$Y_1$ Wasserstoff oder

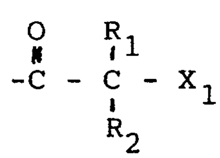

bedeutet, $Y_2$ aus der aus Wasserstoff, Halogen und Nitro bestehenden Gruppe ausgewählt ist,

$R_4$ eine direkte Bindung (-) oder Alkylen mit 1 bis 12 Kohlenstoffatomen ist,

$x_2$ $-O-R_8-O-$ oder

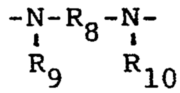

ist, worin:
$R_8$ Alkylen mit 2 bis 12 Kohlenstoffatomen ist,
$R_9$ und $R_{10}$ Alkyl mit 1 bis 18 Kohlenstoffatomen bedeuten oder, zusammen genommen, Alkylen mit 1 bis 12 Kohlenstoffatomen bilden;
und
eine ungesättigte Verbindung mit mindestens einer ethylenischen Doppelbindung, die unter Bildung eines Polymer photopolymerisierbar ist.

2. Photopolymerisierbare Prepolymer-Zusammensetzung, die einen Photoinitator gemäß Anspruch 1 umfaßt und weiterhin ein sekundäres oder tertiäres Alkyl- oder Hydroxyalkylamin enthält.

3. Verfahren zum Photopolymerisieren einer ungesättigten Verbindung mit mindestens einer photopolymerisierbaren, ethylenischen Doppelbindung, das das Initiieren der ungesättigten Verbindung mit aktinischem Licht in Anwesenheit eines Photoinitiators gemäß Definition in Anspruch 1 umfaßt.

4. Carbazol-Photoinitiatoren zur Photopolymerisation von ungesättigten Verbindungen mit einer der Formeln:

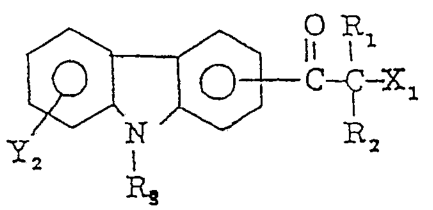

(I)

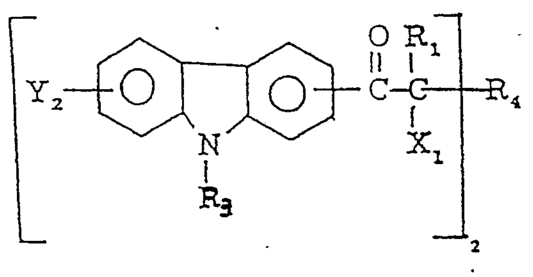

(II).

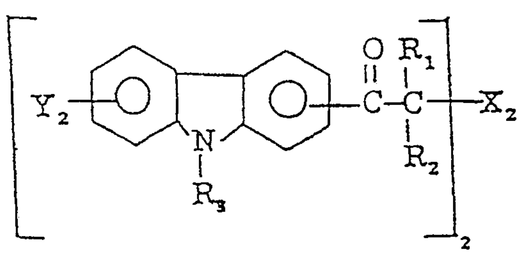

(III)

worin
$R_1$ und $R_2$ jeweils Alkyl mit 1 bis 18 Kohlenstoffatomen bedeuten oder, zusammen genommen, Alkylen mit 2 bis 12 Kohlenstoffatomen bilden,
$R_3$ aus der aus Wasserstoff, Alkyl mit 1 bis 18 Kohlenstoffatomen und Acyl mit 2 bis 12 Kohlenstoffatomen bestehenden Gruppe ausgewählt ist,
$X_1$ $-OR_5$ oder

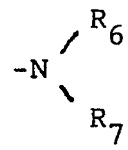

ist, worin $R_5$ aus der aus Wasserstoff Alkyl und Alkenyl mit 1 bis 18 Kohlenstoffatomen bestehenden Gruppe ausgewählt ist und $R_6$ und $R_7$ aus der aus Alkyl, Hydroxyalkyl mit 1 bis 18 Kohlenstoffatomen bestehenden Gruppe ausgewählt sind, und $R_6$ und $R_7$, zusammen genommen, Alkylen mit 2 bis 12 Kohlenstoffatomen und Oxadialkylen und Iminodialkylen mit 4 bis 24 Kohlenstoffatomen bilden,
$Y_2$ aus der aus Wasserstoff, Halogen und Nitro bestehenden Gruppe ausgewählt ist,
$R_4$ eine direkte Bindung (-) oder Alkylen mit 1 bis 12 Kohlenstoffatomen ist,
$X_2$ -O-$R_8$ -O- oder

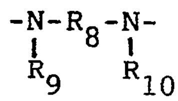

ist, worin:
$R_8$ Alkylen mit 2 bis 12 Kohlenstoffatomen ist,
$R_9$ und $R_{10}$ Alkyl mit 1 bis 18 Kohlenstoffatomen bedeuten oder, zusammen genommen, Alkylen mit 1 bis 12 Kohlenstoffatomen bilden.

5. Carbazol-Photoinitiatoren gemäß Anspruch 4, in welchen $R_1$ und $R_2$ jeweils Alkyl mit 1 bis 18 Kohlenstoffatomen bedeuten.

6. Carbazol-Photoinitiatoren gemäß Anspruch 4, in welchen $R_1$ und $R_2$ zusammen Alkylen mit 2 bis 12 Kohlenstoffatomen bedeuten.

7. Carbazol-Photoinitiatoren gemäß Anspruch 4, in welchen $R_3$ Wasserstoff bedeutet.

8. Carbazol-Photoinitiatoren gemäß Anspruch 4, in welchen $R_3$ Alkyl mit 1 bis 18 Kohlenstoffatomen bedeutet.

9. Carbazol-Photoinitiatoren gemäß Anspruch 4, in welchen $R_3$ Acyl mit 2 bis 12 Kohlenstoffatomen bedeutet.